Europäisches Patentamt

European Patent Office

Office européen des brevets

(19)

(11) Veröffentlichungsnummer: **0 067 410**
**B1**

---

(12) ## EUROPÄISCHE PATENTSCHRIFT

(45) Veröffentlichungstag der Patentschrift:
16.01.85

(51) Int. Cl.⁴: **C 07 C  69/716**, C 07 C  67/31

(21) Anmeldenummer: **82105033.3**

(22) Anmeldetag: **08.06.82**

---

(54) 4-Hydroxyacetessigsäurealkylester und Verfahren zu ihrer Herstellung.

---

(30) Priorität: **17.06.81  CH 3984/81**

(43) Veröffentlichungstag der Anmeldung:
**22.12.82 Patentblatt 82/51**

(45) Bekanntmachung des Hinweises auf die Patenterteilung:
**16.01.85 Patentblatt 85/3**

(84) Benannte Vertragsstaaten:
**AT BE CH DE FR GB IT LI NL SE**

(56) Entgegenhaltungen:
**GB - A - 1 345 081**

**THE JOURNAL OF THE CHEMICAL SOCIETY,
CHEMICAL COMMUNICATIONS, Nr. 24, 21. Dezember
1977, The Royal Society of Chemistry, London (GB), C.B.
TROOSTWIJK et al.: "Method for the synthesis of
4-substituted acetoacetates", Seiten 932-933**

**Die Akte enthält technische Angaben, die nach dem
Eingang der Anmeldung eingereicht wurden und die
nicht in dieser Patentschrift enthalten sind.**

(73) Patentinhaber: **LONZA AG, Gampel/Wallis (CH)**

(72) Erfinder: **Miller, Raimund, Dr., Berkshire Place 66,
Hackensack. N.J. 07601 (US)**
Erfinder: **Tenud, Leander, Dr., Balfrinstrasse 23, Visp
(Kanton Wallis) (CH)**

(74) Vertreter: **Weinhold, Peter, Dr. et al, Siegfriedstrasse 8,
D-8000 München 40 (DE)**

---

Anmerkung: Innerhalb von neun Monaten nach der Bekanntmachung des Hinweises auf die Erteilung des europäischen
Patents im Europäischen Patentblatt kann jedermann beim Europäischen Patentamt gegen das erteilte europäische Patent
Einspruch einlegen. Der Einspruch ist schriftlich einzureichen und zu begründen. Er gilt erst als eingelegt, wenn die
Einspruchsgebühr entrichtet worden ist (Art. 99(1) Europäisches Patentübereinkommen).

## Beschreibung

Die Erfindung betrifft die neuen Verbindungen 4-Hydroxyacetessigsäurealkylester, insbesondere 4-Hydroxyacetessigsäuremethylester, und 4-Hydroxyacetessigsäureäthylester, die unter anderem zur Herstellung von Tetronsäure verwendet werden können.

In der GB-A Nr. 1345081 der Anmelderin ist die Herstellung von 4-Alkoxyacetessigsäureestern durch Umsetzung von 4-Halogenacetessigsäureestern mit den entsprechenden Alkalimetallalkoholaten beschrieben. Des weiteren ist es aus „J.C.S. Chem. Comm." 1977, S. 932/933, bekannt, 4-Bromacetessigsäureäthylester mit Natriumhydrid zu behandeln, um durch anschliessende Umsetzung des erhaltenen Chelates mit Nukleophilen, wie z.B. Alkoholaten, zu z.B. 4-Alkoxyacetessigsäureäthylestern zu gelangen. Ein vorteilhafter Weg zu 5-Hydroxyacetessigsäureestern ist bisher jedoch nicht bekannt.

Wie gefunden wurde, können diese neuen Verbindungen, ausgehend von 4-Halogenacetessigsäurealkylester, über den 4-Benzyloxyacetessigsäurealkylester durch Hydrogenolyse hergestellt werden.

Eine bevorzugte Verfahrensweise liegt darin, dass man 4-Halogenacetessigester mit einem Alkalisalz des Benzylalkohols bei Temperaturen von 0 bis 40°C in einem organischen Lösungsmittel, vorzugsweise Dimethylsulfoxid oder Tetrahydrofuran, umsetzt und den dabei erhaltenen 4-Benzyloxyacetessigsäurealkylester hydrogenolysiert.

Als 4-Halogenacetessigester können die Brom- und Chlorderivate eingesetzt werden. Vorzugsweise geht man von 4-Chloracetessigsäurealkylester aus. Bevorzugt werden im Esterteil $C_1$- bis $C_6$-Alkylgruppen, insbesondere die Methyl- und Äthylgruppe.

Als Alkalisalze des Benzylakohols können die Kalium- oder Natriumsalze Verwendung finden. Vorzugsweise verwendet man das Natriumsalz. Dieses wird vorzugsweise aus Natriumhydrid und Benzylalkohol in einem organischen Lösungsmittel hergestellt. Die Reaktionstemperatur liegt zweckmässig bei 0 bis 40°C.

Unter Hydrogenolyse ist die Spaltung einer Verbindung durch Wasserstoff zu verstehen. Diese Reaktion wird im vorliegenden Fall vorzugsweise unter einem Druck von 1 bis 8 atm durchgeführt. Vorzugsweise wird die Wasserstoffbehandlung in Gegenwart eines Hydrierungskatalysators, wie Edelmetall, z.B. Platin, Ruthenium, Rhodium oder Raney-Nickel, Kobalt und andere mehr, durchgeführt, wobei ein inertes Lösungsmittel für die Acetessigesterverbindung verwendet werden kann. Zur Erhöhung der reagierenden Oberfläche sind diese Katalysatoren zweckmässig auf Träger, z.B. Bimsstein, Kohle, Silicagel, Tonerde und andere mehr, aufgebracht.

*Beispiel 1:*

7,56 g 80%iges Natriumhydrid wurden durch dreimaliges Waschen mit je 30 ml Petroläther (Kp. 40 bis 60°C) von Weissöl befreit und zu 160 ml Tetrahydrofuran gegeben. Unter Rühren wurden nun 14,28 g Benzylalkohol so zudosiert, dass eine Reaktionstemperatur von 40°C eingehalten wurde. Nach beendeter Wasserstoffentwicklung wurde während 1 h eine Lösung von 19,74 g (95,5%ig = 0,1145 mol) 4-Chloracetessigester in 80 ml Tetrahydrofuran zugetropft, wobei eine Temperatur von 40°C herrschte. Nach 15stündigem Rühren, wobei die Temperatur Raumtemperatur annahm, wurde die Hälfte des Tetrahydrofurans am Vakuumrotationsverdampfer abgedampft und der noch fliessbare Rückstand in dünnem Strahl in eine Mischung von 14,3 g konz. HCl in 150 g Eiswasser gegossen, wobei sich der pH nach Ende der Zugabe auf 5 einstellte. Nun wurde 4mal mit Äther extrahiert und nach Waschen und Trocknen der Äther am Vakuumrotationsverdampfer abgedampft. Der Rückstand wurde destilliert.

Es resultierten 22,52 g (83,2%) 4-Benzyloxyacetessigsäureäthylester (Kp.$_{0,4}$ 126°C).

21,24 g dieses Esters wurden, gelöst in Essigester (100 ml), in einen 200-ml-Stahlautoklaven, mit Magnetrührer versehen, eingebracht und in Gegenwart von 1,05 g Pd 5% auf Kohle mit Wasserstoff bei einem Druck von 5 atm hydrogenolysiert. Nach beendeter Reaktion, die etwa 2h betrug, und nach einer 2stündigen Nachreaktionszeit wurde der Katalysator abfiltriert und das Filtrat am Vakuumrotationsverdampfer bei 35°C eingedampft. Der Rückstand wurde im Hochvakuum getrocknet. Es resultierten 13,4 g 4-Hydroxyacetessigsäureäthylester in Form eines leicht gelblichen Öles.

*Elementaranalyse für $C_6H_{10}O_4$ (MG 146,14)*

Gefunden:  C 49,7% $\pm$ 0,1   H 7,1% $\pm$ 0,1
Berechnet:  C 49,3%        H 6,9%

*IR (dünner Film)*

Banden bei 3480(vs), 2950(m), 1745(vs), 1725(vs), 1325(m), 1030(m) cm$^{-1}$

*NMR (10% in $CDCl_3$)*

$\delta$ = 1,30 (t, 3H)
$\delta$ = 3,52 (s, 2H)
$\delta$ = 4,27 (q) (4H)
$\delta$ = 4,10 (s) (4H)
OH bei $\delta$ = 3,2-3,7 ppm
*MS:* M$^+$ 164, M/e 115, 101, 88.

*Beispiel 2:*

22,68 g 80%iges Natriumhydrid wurden analog Beispiel 1 mit Petroläther gewaschen und zu 460 ml Tetrahydrofuran gegeben. Unter Rühren wurden innert 45 min 42,84 g Benzylalkohol bei 20 bis 32°C zugetropft. Nachdem die $H_2$-Entwicklung aufgehört hat, wurden in 45 min bei 20 bis 30°C 54,2 g (97,5%ig = 0,35 mol) 4-Chloracetessigsäuremethylester zugetropft. Die hellbraune Suspension wurde dann 4 h bei ca. 20°C gerührt und anschliessend auf eine Lösung von 92,9 g konz. Salzsäure in 400 ml Wasser gegossen. Die hellbraune Mischung wurde mit 3 Portionen Äther zu je 150 ml extrahiert, die Extrakte vereinigt und zweimal mit je 100 ml gesättigter NaCl-Lösung gewaschen. Die Ätherphase wurde über $Na_2SO_4$ getrocknet, filtriert und im Rotationsverdampfer bei 30 bis 40°C eingedampft. Der Rückstand wurde destilliert und es resultierten 45,1 g (58,1%) 4-Benzyloxyacetessigsäuremethylester (Kp. 1 mm = 126 bis 129°C).

*IR (dünner Film)*

Banden bei 1752(vs), 1730(vs), 1325(m), 1100(m) cm$^{-1}$

*NMR (DMSO-d6)*
δ = 7,32 (m, 5H)
δ = 4,51 (s, 2H)
δ = 4,25 (s, 2H)
δ = 3,62 (s, 3H)
δ = 3,60 (s, 2H)

22,22 g dieses Esters, in 100 ml Essigester gelöst, wurden in einem 200-ml-Stahlautoklaven, mit Magnetrührer versehen, eingebracht und in Gegenwart von 1,1 g Pd 5% auf Kohle mit Wasserstoff bei einem Druck von 5 bar hydrogenolysiert. Nach der Reaktion wurde der Katalysator abfiltriert und das Filtrat im Rotationsverdampfer bei 30 bis 35°C eingedampft. Der hellbraune Rückstand wurde noch 1 h im Hochvakuum weitergetrocknet. Als Produkt erhielt man 13,3 g 4-Hydroxyacetessigsäuremethylester in Form einer gelben Flüssigkeit.

*IR (dünner Film)*

Banden bei 3460(m), 1750(vs), 1730(vs), 1440(m), 1330(m), 1270(m), 1060(m) cm$^{-1}$

*NMR (DMSO-d6)*
δ = 5,00 (breit, 1+H)
δ = 4,10 (s, 2H)
δ = 3,60 (s, 3H)
δ = 3,54 (s, 2H)

## Patentansprüche

1. 4-Hydroxyacetessigsäurealkylester.

2. 4-Hydroxyacetessigsäuremethylester.
3. 4-Hydroxyacetessigsäureäthylester.
4. Verfahren zur Herstellung von 4-Hydroxyacetessigsäurealkylester gemäss Patentanspruch 1, dadurch gekennzeichnet, dass man 4-Halogenacetessigsäurealkylester in den 4-Benzyloxyacetessigsäurealkylester überführt und letzteren hydrogenolysiert.
5. Verfahren gemäss Patentanspruch 4, dadurch gekennzeichnet, dass man die Hydrogenolyse unter Druck und in Gegenwart von Hydrierungskatalysatoren durchführt.

## Claims

1. Alkylesters of 4-hydroxy-acetoacetic acid.
2. Methylester of 4-hydroxy-acetoacetic acid.
3. Ethylester of 4-hydroxy-acetoacetic acid.
4. Process for the preparation of alkylesters of 4-hydroxyacetoacetic acid according to Claim 1, characterized in converting alkylesters of 4-halo-acetoacetic acid into alkylesters of 4-benzyloxy-acetoacetic acid and hydrogenolyzing the latter.
5. Process according to Claim 4, characterized in that the hydrogenolysis is carried out under pressure and in the presence of hydrogenation catalysts.

## Revendications

1. 4-Hydroxyacétylacétates d'alcoyle.
2. 4-Hydroxyacétylacétate de méthyle.
3. 4-Hydroxyacétylacétate d'éthyle.
4. Procédé pour la préparation de 4-hydroxyacétylacétates d'alcoyle selon la revendication 1, caractérisé en ce qu'on transforme un 4-halogénoacétylacétate d'alcoyle en le 4-benzyloxyacétylacétate d'alcoyle et hydrogénolyse ce dernier.
5. Procédé selon la revendication 4, caractérisé en ce qu'on effectue l'hydrogénolyse sous pression et en présence de catalyseurs d'hydrogénation.